# EUROPEAN PATENT SPECIFICATION

(11) **EP 2 914 341 B1**
(45) Date of publication and mention of the grant of the patent: **15.04.2020**
(21) Application number: 13795909.4
(22) Date of filing: 05.11.2013
(51) Int. Cl.: A61N 7/00, A61B 8/00

(54) **NON-INVASIVE LUNG PACING**
NICHTINVASIVE LUNGENSTIMULATION
STIMULATION PULMONAIRE NON INVASIVE

(30) Priority: 05.11.2012 US 201261722596 P
(43) Date of publication of application: 09.09.2015
(73) Proprietor: Regents of the University of Minnesota, Minneapolis, MN 55455 (US); Paul, Saurav, Minneapolis, Minnesota 55414 (US)
(72) Inventor: CABRERA, Jesus Arturo, Minneapolis, Minnesota 55410 (US); BALLARD, John R., St. Bonifacius, Minnesota 55375 (US); KROCAK, James C., Minneapolis, MN 55401 (US); PAUL, Saurav, Shoreview, Minnesota 55126 (US)
(74) Representative: Parker, Andrew James
(86) International application number: PCT/US2013/068538
(87) International publication number: WO 2014/071386

(56) References cited:
- US-A1- 2006 052 695
- US-A1- 2008 046 053
- US-A1- 2010 094 149
- US-A1- 2010 113 928
- US-A1- 2011 029 044
- US-A1- 2011 130 615
- US-A1- 2013 281 890
- US-B1- 7 340 302

## Description

### BACKGROUND

Controlled mechanical ventilation (CMV) is a mode of mechanical ventilation (MV) in which the ventilator provides the work of breathing. Generally, MV can sustain pulmonary gas exchange in patients who are incapable of maintaining sufficient alveolar ventilation, or that are having difficulties with oxygenation and/or removing carbon dioxide from their blood, in particular while in the intensive care unit (ICU). Some indications for MV include respiratory failure due to chronic obstructive pulmonary disease, status asthmaticus, heart failure, neuromuscular diseases, drug overdoses, upper cervical spinal injury, and/or during postsurgical recovery. Over time, and as the patient's underlying disease state is treated, the patient will be placed on ventilation modes that allow the patient to gain more control of their breathing pattern (respiratory rate) as well as the depth of their breaths (tidal volume), with the ventilator assisting respiration cycles that the patient cannot support. As the patient's respiratory drive strengthens, the patient is evaluated for extubation with trials of minimal or no ventilator support, in a test known as the Spontaneous Breathe Test. With favorable breathe test results and the patient demonstrating sufficient strength to breathe on their own, the individual will ultimately be liberated from the ventilator.

Prolonged MV is associated with complications such as tracheal injuries, infection, cardiovascular failure, and lung injury. Prolonged MV may also lead to diaphragmatic weakness due to both atrophy and contractile dysfunction, resulting in a condition that has been termed ventilator-induced diaphragmatic dysfunction, herein VIDD. VIDD makes it difficult to remove a patient from MV support. Often, the patient becomes quickly exhausted from natural breathing or they cannot breathe at all. Consequently, patients suffering from VIDD must be "weaned" from MV, which is a process that alternates the patient between ventilator support and natural breathing. The attempted natural breathing helps to increase diaphragmatic strength, such that the patient can ultimately breathe without MV support. Weaning can substantially increase the length of ICU stay, and consequently increase the risk of severe MV complications, including ventilator-associated pneumonia and ventilator-induced lung injury.

Prolonged CMV can result in rapid onset of diaphragmatic atrophy compared to locomotor skeletal muscles during periods of disuse. In humans, CMV can result in atrophy of the human diaphragm in times as short as 18-69 hours.

US2011/0029044 discloses an implantable stimulator for stimulation of the phrenic nerve.

### OVERVIEW

The present inventors have recognized, among other things, that diaphragmatic atrophy can be treated using phrenic nerve stimulation. Stimulation of the phrenic nerve can reverse or stop VIDD. Intervention can reduce the length of the weaning period and ICU stay.

One approach can include transdermally stimulating a phrenic nerve to cause diaphragmatic action. An external sensor is non-invasive and can provide a measure of diaphragmatic action and a processor can execute a set of instructions for modulating a parameter corresponding to a measure of assistance provided by the stimulation. The processor can be configured to wean a patient from reliance on external stimulation and promote autonomous breathing without continuous aid from a stimulator and without the aid of a mechanical ventilator.

An example of the present subject matter includes acoustic stimulation of the phrenic nerve. Non-invasive neuromodulation utilizes acoustic energy to stimulate the phrenic nerve, thereby treating ventilator-induced diaphragmatic dysfunction (VIDD). Stimulation of the phrenic nerves can increase diaphragmatic strength and prevent diaphragm atrophy.

Ultrasonic stimulation can modulate neural activity under various conditions. Low-Intensity, Low-Frequency Ultrasound is capable of stimulating action potentials and synaptic transmission in neural tissue for non-peripheral nerves, as is the use of Low-Intensity Focused Ultrasound.

According to one theory, the mechanisms governing ultrasound-mediated modulation of neural activity may be the production of local membrane depolarization that activates voltage-gated sodium channels or, alternatively, the induced conformational changes in protein structure that may modulate ion flux into and out of the cell. The hypothesized mode of action of acoustic neuromodulation is temporary and reversible poration of the nerve fibers, which leads to cellular flux of Na⁺, Ca²⁺, and K⁺ ions, which translates into an electronic gradient that resembles the electric impulse and the action potential that is either naturally generated and/or provided via electric stimulation. Acoustic stimulation is effectively non-thermal and the acoustic energy delivery to stimulate the phrenic nerve, in terms of energy per unit time, is below the threshold for ultrasound imaging.

Ultrasound energy can induce nerve stimulation both in the central nervous system and the peripheral nervous system. For example, an ultrasound probe at a frequency of 350 kHz, can activate the abducens nerve, a peripheral nerve, and obtain abductive eye movement. Stimulation success rates will vary based on intensity levels and frequencies. For example, neuronal activity success rates of transcranial stimulation can increase with decreased intensity at lower frequencies. For example, using a 350 kHz transducer, abductive eye movement can be successfully elicited whereas a 500 kHz transducer at the same intensity is ineffective.

The problem of prolonged ventilation and associated diaphragmatic atrophy can be addressed by a non-invasive neuromodulation platform that can stimulate the phrenic nerves, as well as other nerves throughout the body, without an interventional procedure.

One example of a solution includes a system that can be configured to deliver energy, spanning several parameters, including ultrasonic frequencies ranging from 100 kHz to 10 MHz, acoustic pressure up to 5 MPa, and a pulse repetition frequency (PRF) up to 100 Hz. The waveform can be a single frequency, a modulated sinusoidal waveform, or an arbitrary waveform.

This overview is intended to provide an overview of subject matter of the present patent application. It is not intended to provide an exclusive or exhaustive explanation of the invention. The detailed description is included to provide further information about the present patent application. The invention is defined in appended independent claim 1, preferred embodiments are described in the dependent claims.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 includes a block diagram of a system, according to one example.
FIG. 2A includes a diagram of a focused ultrasonic transducer and a phrenic nerve, according to one example.
FIG. 2B includes a diagram of an unfocused ultrasonic transducer and a phrenic nerve, according to one example.
FIG. 3 includes a diagram of an electrode needle and a transducer, according to one example.
FIGS. 4A, 4B, and 4C illustrate views of multiple element ultrasonic transducers, according to various examples.
FIG. 5 includes a view of a sensor and a belt, according to one example.
FIG. 6 includes a view of a patient fitted with a transducer, a therapy device, and a sensor, according to one example.
FIG. 7 illustrates ultrasonic stimulation of a phrenic nerve along various axes, according to various examples.
FIGS. 8A and 8B illustrate a rotatably adjustable transducer, according to one example.
FIG. 9 illustrates a duty cycle, according to one example.
FIG. 10 illustrates a method, according to one example.

### DETAILED DESCRIPTION

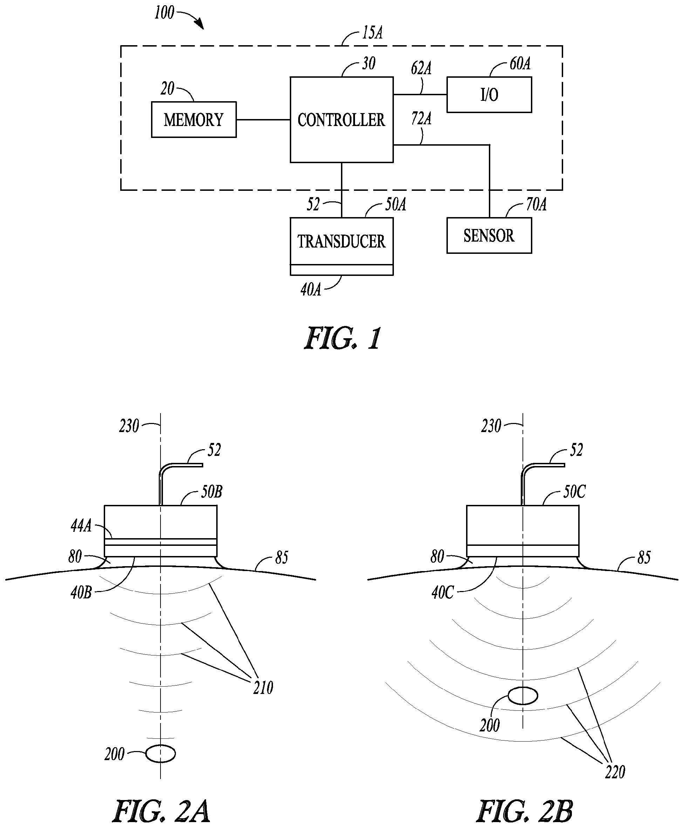

FIG. 1 includes a block diagram view of system 100, according to one example. System 100, in the example shown, includes therapy device 15A, transducer 50A, and sensor 70A. Therapy device 15A includes controller 30, memory 20, and I/O module 60A.

Controller 30 can include a digital circuit, an analog circuit, or a combination of digital and analog circuitry. In one example, controller 30 includes a processor configured to execute an algorithm. Controller 30 can include an output driver, a filter, an amplifier, a signal generator, an impedance matching circuit, or other circuit to adjust or condition a signal level provided to transducer 50A (via link 52). Controller 30 is also configured to read stored data or instructions in memory 20, write data to memory 20, exchange data with I/O module 60A and exchange data with sensor 70A. Controller 30 can also be referred to as a control module.

Memory 20 can include a digital storage device or an analog storage device. Memory 20 can provide storage for instructions for execution by a processor of controller 30. Memory 20 can provide storage for data and calculated results.

I/O module 60A can include an input module, an output module, or both an input and an output module. An example of an input module can include a keyboard, a mouse, a touch-screen, a microphone, a network interface, or other input device. An example of an output module can include a printer, visible display, a speaker, or other circuit. In the figure, I/O module 60A is shown coupled to controller 30 by link 62A. Link 62A can include a wired or wireless connection. In one example, I/O module 60A allows user-controlled selection of a parameter for excitation. As used in this document, a link can include a wired communication channel (such as an electrical wire) or a wireless communication channel (such as a fiber-optic element, infrared channel, or other type of channel).

Sensor 70A provides an electrical output signal (on link 72A) based on a measured or sensed condition. For example, sensor 70A can include a patch-type sensor such as a piezoelectric sensor, an accelerometer, a pressure sensor, or a microphone configured to detect a hiccup condition of a patient. Sensor 70A can include a magnetoresistive sensor or other device that generates an electrical output in response to an ultrasonic or audible signal. Link 72A can be wired or wireless.

Transducer 50A can include an ultrasonic transducer. In various examples, transducer 50A includes a non-implantable transducer configured to generate an ultrasonic output in response to a driving signal provided by controller 30 and conveyed to transducer 50A by link 52. Ultrasonic energy, in the form of mechanical vibrations, is applied to the surface of tissue by emitting surface 40A. Emitting surface 40A can be in direct physical contact with the tissue, can be positioned proximate the tissue, or can be acoustically coupled to the tissue by a coupling medium. Transducer 50A is configured to emit a longitudinal wave at an ultrasonic frequency in response to electrical energy. The emitted wave is aligned generally normal with an emitting surface and in various examples, transducer 50A can have a single emitting element or can have multiple emitting elements. In one example, transducer 50A has a diameter of approximately 25.4 mm and can be referred to as a patch.

All or some components of system 100 can be disposed in a common housing or selected components of system 100 can be in various housings with suitable interconnects or communication channels there between.

FIG. 2A includes a diagram of ultrasonic transducer 50B and phrenic nerve 200, according to one example. In this example, transducer 50B emits focused energy aligned on axis 230 and passing normal to emitting surface 40B, as denoted by waves 210. As shown, waves 210 are transmitted transdermally into tissue 85 and pass through phrenic nerve 200, shown here aligned on axis 230. Transducer 50B emits ultrasonic energy in response to an electrical driving signal provided on link 52. In the example shown, coupling medium 80 is disposed between emitting surface 40B of transducer 50B and tissue 85 and enables efficient communication of energy from transducer 50B to tissue 85.

Coupling medium 80 can include a gel, a plastic element, or an air gap. In this example, transducer 50B is fitted with lens 44A which focuses the emitted ultrasonic energy. Coupling medium 80 can provide electrical insulation and acoustic coupling.

In one example, transducer 50B is positioned in the neck region, on the upper torso, or over the anterior scalene muscle and is configured to deliver an acoustic impulse that affects the phrenic nerve. Phrenic nerve 200, as shown herein, is illustrated as an oval shape however, it will be understood that the phrenic nerve in a human refers to tissue that follows a path through the neck.

FIG. 2B includes a diagram of ultrasonic transducer 50C and phrenic nerve 200, according to one example. In this example, transducer 50C emits unfocused energy aligned on axis 230 and passing normal to emitting surface 40C, as denoted by waves 220. As shown, waves 220 are transmitted transdermally into tissue 85 and pass through phrenic nerve 200, also aligned on axis 230. Transducer 50C emits ultrasonic energy in response to an electrical driving signal provided on link 52. Coupling medium 80 is disposed between emitting surface 40C of transducer 50C and tissue 85 and enables efficient communication of energy from transducer 50C to tissue 85.

Transducer 50B and transducer 50C are shown here in profile view and can each have a cross-section that is round, rectangular, or any other shape.

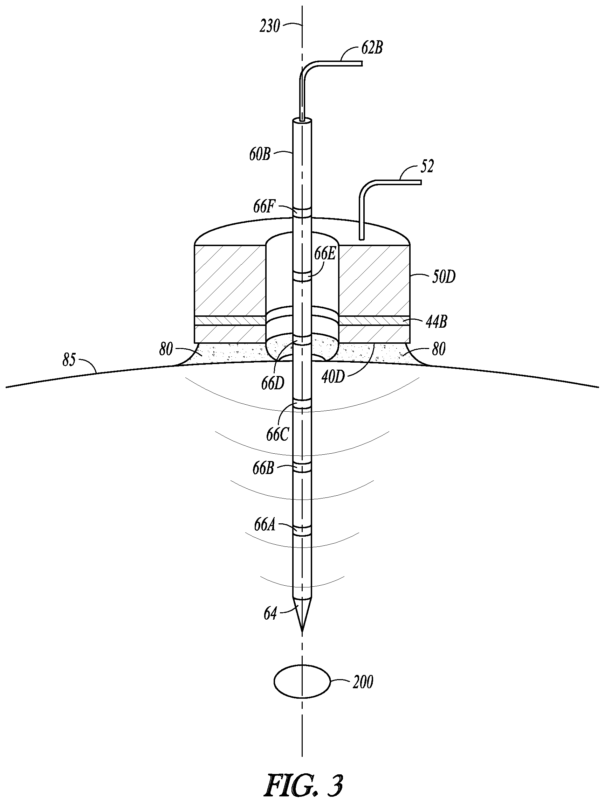

FIG. 3 includes a diagram needle 60B and transducer 50D, according to one example. In this example, transducer 50D emits a focused ultrasonic wave aligned on axis 230. Transducer 50D, and thus the emitted wave, are shown here as aligned on phrenic nerve 200. In this example, the emitted wave is shaped by focus lens 44B affixed near emitting surface 40D of transducer 50D. Transducer 50D emits ultrasonic energy based on an electrical signal provided on link 52. Acoustical energy provided by transducer 50D is communicated to tissue 85 by coupling medium 80. In this example, transducer 50D has an open center configuration in which emitting surface 40D has an annular ring arrangement.

In the example shown, needle 60B is aligned coaxially on axis 230 and freely passes through the open center of transducer 50D. In one example, needle 60B includes an electrically non-conductive body diameter along which are disposed a plurality of insertion depth marks, here shown as 66A, 66B, 66C, 66D, 66E, and 66F.

In the example shown, insertion depth marks 66A, 66B, 66C, 66D, 66E, and 66F include an electrically conductive outer surface. Needle 60B is part of a feedback mechanism that can facilitate placement of transducer 50D. In one example, link 62B includes an electrical conductor that carries an electrical current corresponding to the depth of insertion of needle 60B in tissue 85. The depth of insertion can be determined based on an electrical signal propagated using a conductive tip 64 or depth marks 66A, 66B, 66C, 66D, 66E, and 66F disposed on a surface of needle 60B.

Like I/O (input/output) module 60A, an example of needle 60B is configured to provide a signal for input to controller 30 in response to a sensed condition or parameter and is also configured to provide an output based on a signal received from controller 30. For example, controller 30 can provide an output signal to needle 60B that includes an electric potential provided to tip 64 and configured to stimulate the phrenic nerve and to elicit a measurable hiccup or other change that can be detected by a sensor. In another example, controller 30 can receive an electrical signal generated by needle 60B and corresponding to a depth, for example, of insertion into tissue 85 at the point of tip 64 near phrenic nerve 200. The electrical signal provided by needle 60B can be determined based on the depth of insertion of needle 60B as indicated by an electrical signal corresponding to the number of insertion depth marks that are below a surface of tissue 85. The insertion depth signal can correspond to a measure of capacitance, a measure of resistance (or conductance) or other measurable parameter and based, in some manner, on the number of insertion depth marks that are below (or above) the surface of tissue 85.

In one example, insertion depth marks 66A, 66B, 66C, 66D, 66E, and 66F each include a visible mark for which the operator can observe and take note of the insertion depth.

In one example, phrenic nerve 200 is sufficiently near to tip 64 to provide good alignment. The acoustic signal from the transducer will have an active diameter that is greater than a diameter of the needle such that the phrenic nerve will be activated if positioned within a distance of the active diameter and without regard to the orientation or geometry of the needle. The needle serves as an electrical stimulation (e-stim) probe that can function to direct the ultrasonic energy. The e-stim signal will propagate spherically around the electrical members and will stimulate the phrenic nerve if located within the sphere. The active diameter of the acoustic signal can be paired such that the active acoustic diameter effectively matches, or is slightly greater than the e-stim sphere. The active acoustic diameter (which can include a columnar acoustic beam) can be correlated with the diameter of the spherical electrical impulse.

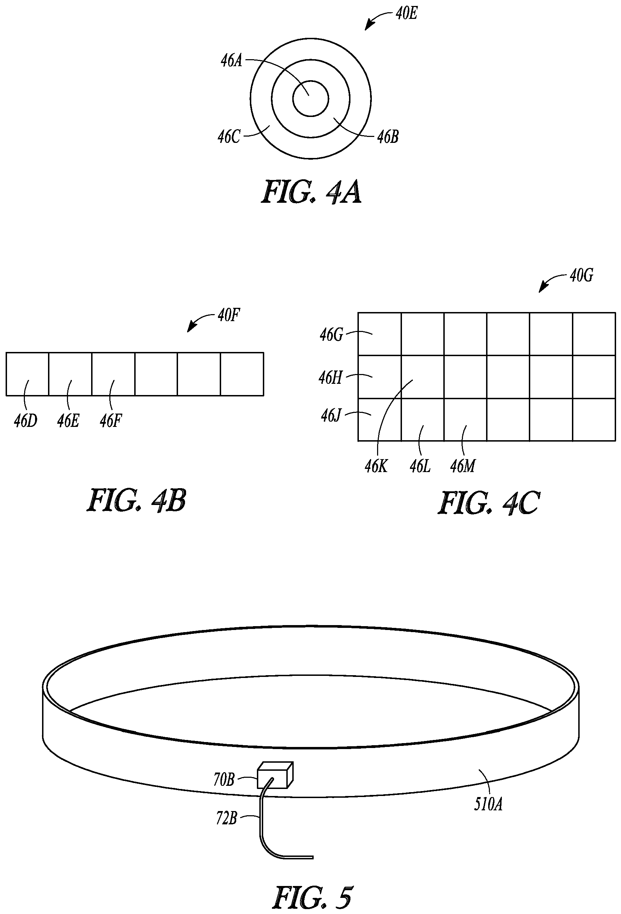

FIGS. 4A, 4B, and 4C illustrate views of emitting surfaces for multiple element transducers, for example, such as transducers 50A, 50B, or 50C. In FIG. 4A, emitting surface 40E corresponds to a transducer having a circular profile and, in the example shown, includes concentric elements 46A, 46B, and 46C arranged in an annular array. In one example, each of elements 46A, 46B, and 46C can be independently driven. Accordingly, an electric driving signal provided to a transducer having elements arranged as shown in FIG. 4A can be configured to selectively steer the emitted energy to a target having a particular depth. The transducer can be physically manipulated to manually align with a target, such as the phrenic nerve, and the ultrasonic energy excitation depth can be selected by manipulating the phase, amplitude, timing, and frequency of excitation signals provided to each of elements 46A, 46B, and 46C.

In FIG. 4B, emitting surface 40F corresponds to a transducer having a rectangular profile and, in the example shown, includes a linear array of six elements, some of which are labeled as elements 46D, 46E, and 46F. In one example, each of elements 46D, 46E, and 46F can be independently driven. Accordingly, an electric driving signal provided to a transducer having elements arranged as shown in FIG. 4B can be configured to selectively steer the emitted energy to a target having a particular depth and having a particular alignment along the length of the transducer. The transducer can be physically manipulated to manually align with a target, such as the phrenic nerve, and the ultrasonic energy excitation depth can be selected by manipulating the phase, amplitude, timing, and frequency of excitation signals provided to each of elements 46D, 46E, and 46F.

In FIG. 4C, emitting surface 40G corresponds to a transducer having a rectangular profile and, in the example shown, includes a 3x6 matrix of elements, some of which are marked as elements 46G, 46H, 46J, 46K, 46L, and 46M. In one example, each of elements 46G, 46H, 46J, 46K, 46L, and 46M can be independently driven. Accordingly, an electric driving signal provided to a transducer having elements arranged as shown in FIG. 4C can be configured to selectively steer the emitted energy to a target having a particular depth, having a particular alignment along the length of the transducer, and having a particular alignment along the height of the transducer. In addition, the transducer can be physically manipulated to manually align with a target, such as the phrenic nerve, and the ultrasonic energy excitation depth can be selected based on manipulating the phase, amplitude, timing, and frequency of excitation signals provided to each of elements 46G, 46H, 46J, 46K, 46L, and 46M. A transducer can have a greater number of elements or a fewer number of elements than that shown in the figures.

The elements of a multi-element transducer can be separately or collectively controlled and activated. For example, a three element transducer can be coupled to controller 30 (FIG. 1) by three independent electronic pathways for separately controlling or operating each element.

In addition, a multi-element transducer can have a planar or curved structure that positions the elements for focused energy delivery. In one example, the transducer has a flexible structure that allows the elements to conform to a contour of the tissue site.

FIG. 5 includes a view of sensor 70B and belt 510A, according to one example. Sensor 70B can include an accelerometer, a microphone, a pressure sensor, a position sensor, or other device that provides an electrical output, carried using link 72B, based on a signal provide by sensor 70B. Sensor 70B can be configured to detect diaphragm contraction and is affixed to belt 510A, which can be positioned about a portion of a patient.

Sensor 70B can be included within a belt, as shown. Sensor 70B can also be included in a patch, a sticker, or other configuration that can secure the device in a relatively fixed position on the patient's surface, such that minor alteration in diaphragmatic response can be detected by the sensor. Additional sensors 70B can also be used, such as a sensor that utilizes electrical impedance to assess inflation of the lungs (i.e. filling the lungs with a non-conductive medium).

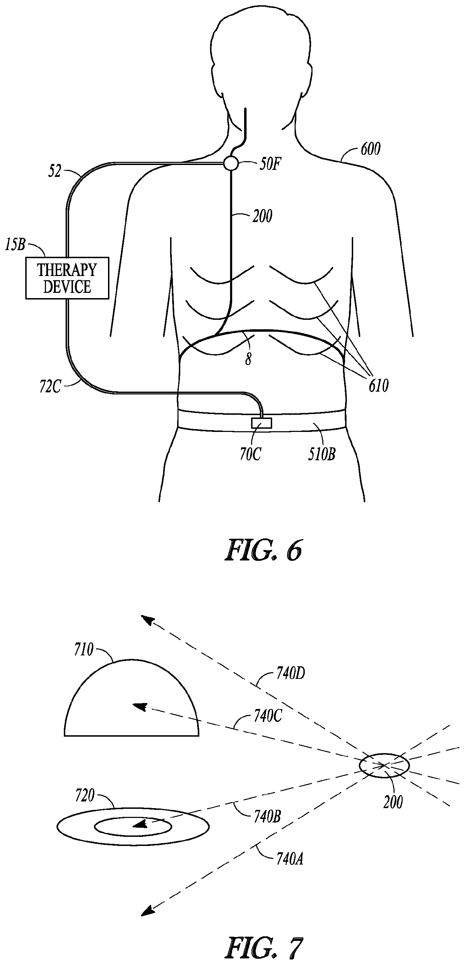

FIG. 6 includes a view of patient 600 fitted with transducer 50F, therapy device 15B, and sensor 70C, according to one example. Phrenic nerve 200 of patient 600 runs from a spinal nerve, through the chest cavity, and to the thoracic diaphragm 8. When positioned for therapy, transducer 50F is located externally and near the neck or upper torso of patient 600 and at a site selected for stimulation of phrenic nerve 200. Transducer 50F is coupled to therapy device 15B by link 52. Therapy device 15B is also coupled to sensor 70C by link 72C. In the example shown, sensor 70C is carried on belt 510B. In this example, sensor 70C, and thus belt 510B, is positioned below the ribcage (as shown at ribs 610).

According to one example, sensor 70C is configured to detect a hiccup associated with movement of thoracic diaphragm 8, and thus signal that ultrasonic stimulation energy is properly targeted and reaching phrenic nerve 200. Sensor 70C can be affixed to patient 600 by an adhesive or affixed by a belt having a buckle or other fastener. In addition, transducer 50F can be affixed by adhesive, a suture, a staple, a magnet, a collar, a neck brace, gauze, a bandage, or a strap or other attachment structure. Therapy device 15B can be portable (battery operated) or line-powered, and can be configured for table-top use, rack mounting, or handheld use.

Sensor 70C need not be included in a belt. In one example, sensor 70C is configured as a sticker or pad that can be temporarily positioned on the patient.

FIG. 7 illustrates ultrasonic stimulation of phrenic nerve 200 using energy delivered along different axes, according to various examples. Each of axis 740A, axis 740B, axis 740C, and axis 740D can be used for stimulating phrenic nerve 200. Axis 740A and axis 740D each represent energy (radiated from a source not shown in the figure) delivered to phrenic nerve 200. The energy represented by axis 740A and by axis 740D is dissipated by other tissue and organs (not shown in this figure). The energy passing beyond phrenic nerve 200 can affect other tissue or nerves, and thus yield an undesirable outcome for the patient. Axis 740B represents energy delivered to phrenic nerve 200 and passing into esophagus 720. Esophagus 720 can include an air filled chamber. The air filled chamber of esophagus 720 can dissipate the ultrasonic energy carried on axis 740B. Axis 740C represents energy delivered to phrenic nerve 200 and passing into trachea 710. Trachea 710 includes an air filled chamber. The air filled chamber of trachea 710 can dissipate the ultrasonic energy carried on axis 740C. Axes 740B and 740C can be viewed as pathways that cross the patient midline and result in ultrasonic energy directed to an air filled void or backstop. In one example, an apical portion of a lung (cupula) serves as the backstop for dissipating energy and in this case, the transducer is aligned to direct energy in a generally downward direction.

Energy routed to the air filled chambers of the trachea or esophagus may dissipate and some energy will be reflected because of the mismatch of the acoustical impedance of tissue and air. According to one example, the axis of stimulation is aligned as shown at axis 740D and axis 740A.

In one example, a transducer is positioned and aligned to engage the phrenic nerve at a site sufficiently remote from other nerves. If the phrenic nerve is not sufficiently separated from other nerves, then ultrasonic stimulation may result in twitching and uncomfortable activation.

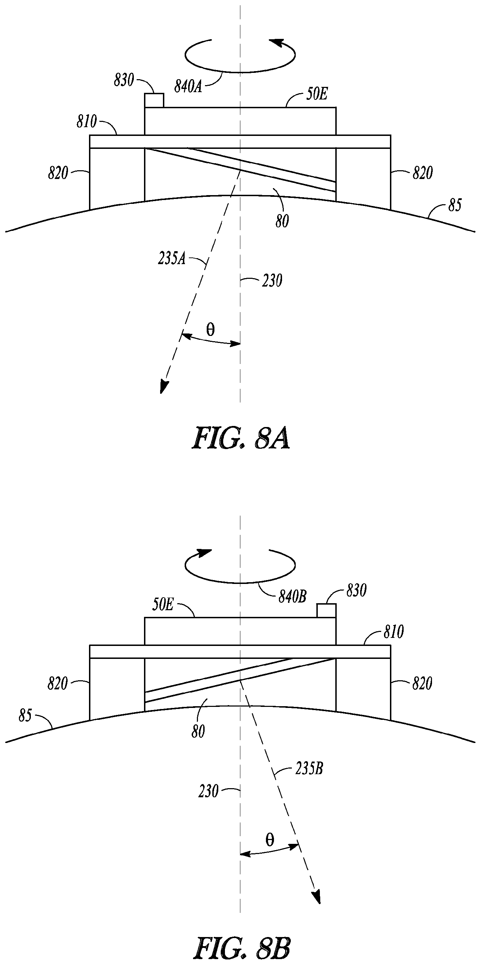

FIGS. 8A and 8B illustrate rotatably adjustable transducer 50E, according to one example. Transducer 50E can have a circular profile, and in the example shown in the figures, corresponds to a circular segment. Transducer 50E includes an attachment flange 810. Attachment flange 810 provides a bonding surface for adhesive 820 by which transducer 50E is affixed to tissue 85 and allows rotation of transducer 50E relative to tissue 85. Transducer 50E has center axis 230 and provides ultrasonic stimulation along an axis displaced from center axis 230 by angle θ and having alignment as shown by stimulation axis 235A (FIG. 8A) and stimulation axis 235B (FIG. 8B). Transducer 50E has a biased emitting surface that interfaces with tissue 85 by way of coupling medium 80. Index mark 830 indicates relative alignment of the stimulation energy and, as shown in FIG. 8A energy is emitted leftward of center axis 230 and with rotation of transducer 50A (about axis 230) as shown by arrow 840A, alignment of the stimulation energy can be altered to a configuration as shown in FIG. 8B in which energy is emitted rightward of center axis 230. Rotation about axis 230, as shown by arrow 840B, returns alignment of the stimulation energy to that shown in FIG. 8A. Transducer 50E can be manipulated to direct energy away from collateral nerves.

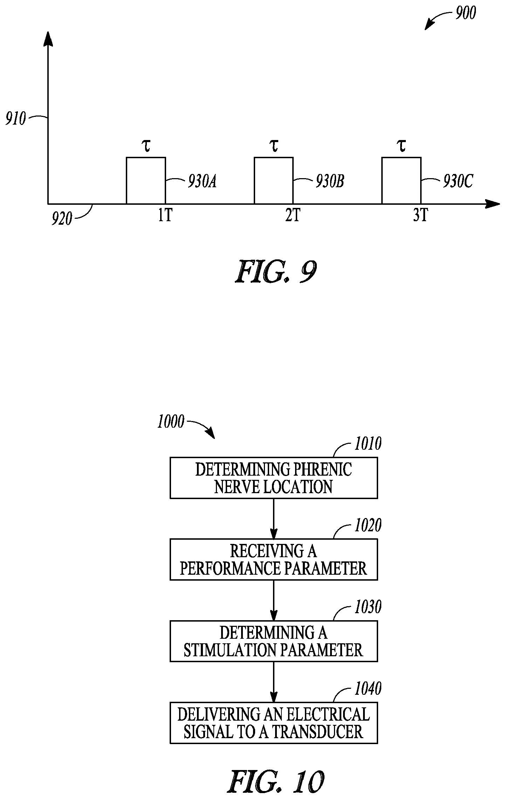

FIG. 9 illustrates timing diagram 900 having a duty cycle according to one example. Timing diagram 900 depicts time on axis 920 and amplitude, for example, on axis 910. Timing diagram 900 can illustrate transdermal stimulation energy provided to a phrenic nerve. In this example, ultrasound energy is provided on a duty cycle expressed as a percentage of τ relative to T. According to one example, during a quiescent period, no stimulation energy is provided and during the τ periods, transdermal ultrasonic simulation is provided to the phrenic nerve. Within each τ there can be several periods (pulse trains) related to the ultrasound signal. The ultrasound frequency is many orders of magnitude higher than the pulse repetition frequency (1/T).

FIG. 10 illustrates method 1000, according to one example. Method 1000 can be implemented by a processor, such as that found in controller 30 (FIG. 1) and executed according to instructions stored in memory (such as memory 20). At 1010, method 1000 includes determining a phrenic nerve location. In one example, the phrenic nerve location is identified using an electronically operated needle (such as needle 60B shown in FIG. 3). For example, needle 60B can be manipulated to bring tip 64 into a position near the phrenic nerve. An electrical signal, when provided to the phrenic nerve by the electrically conductive tip 64, will cause the diaphragm to contract, thereby inducing a hiccup. The hiccup can be detected audibly or by a sensor, such as sensor 70A (FIG. 1) or sensor 70B (FIG. 5). Using needle 60B, depth of insertion can be determined using depth marks 66A, 66B, 66C, 66D, 66E, and 66F (FIG. 3). In one example, the phrenic nerve location is determined by sweeping alignment and depth of penetration of an ultrasonic stimulation signal and monitoring for hiccup. In one example, the ultrasonic stimulation signal can be swept by physically manipulating an ultrasonic transducer. In one example, an array of elements of an ultrasonic transducer can be operated in a controlled manner to discern the location of the phrenic nerve.

At 1020, method 1000 includes receiving a performance parameter. The performance parameter can represent a measure of diaphragmatic activity. For example, the measure of a performance parameter will likely exhibit a gradient between the time immediately following ventilator removal and a time corresponding to fully restored diaphragm activity. For example, a performance parameter can indicate that the patient needs diaphragmatic assist in 20 percent of breath cycles and in 80 percent of breath cycles, no assist is needed. A sensor, such as sensor 70A or 70B, can provide a feedback signal indicative of a performance parameter. In one example, the performance parameter is calculated by a processor or determined by data entered or provided at I/O 60A.

At 1030, method 1000 includes determining a stimulation parameter. The stimulation parameter can correspond to a phase, an amplitude, a time, a frequency, a duty cycle, or other parameter. The stimulation parameter can be encoded data corresponding to delivery of ultrasonic stimulation at, for example, an amplitude of 720 mW/cm² Iₛₚₜₐ spatial-peak temporal-average intensity, a duration of 200 msec, a frequency of 250 kHz, and with a duty cycle of 30 percent. In another example, the stimulation parameter can be encoded as data, or as a signal, corresponding to a phase configuration for an array of elements of a transducer that when operated according to the stimulation parameter, achieves therapeutic stimulation.

At 1040, method 1000 includes delivering an electrical signal to a transducer. The electrical signal is configured to provide ultrasonic stimulation tailored to a therapy regimen. Method 1000 can be repeated until successful stimulation of the phrenic nerve is observed to obtain the desired stimulation parameters for the patient. These parameters then can be utilized for continuous stimulation at the desired PRF in order to have sustained pulsed diaphragmatic activity.

Method 1000 can include processes in addition to delivering an electrical impulse to the transducer. The resulting acoustic impulse can be utilized to stimulate the phrenic nerve.

### Various Notes & Examples

The transducer emitting surface can have any number of elements in any number of configurations. One example includes an array of elements arranged in a narrow strip. The strip can encircle a target area and controller 30 can be configured to activate individual elements in a manner tailored to discern those elements most effective for eliciting a response (as measured by sensor 70A, for example). For example, a wide, plain beam of excitation (unfocused) can be delivered and, by modulating the excitation, a sensor can indicate a change in response at the diaphragm and thus aid in identifying the location of the phrenic nerve.

An algorithm can be configured to select or control a particular duty cycle for excitation. An example of a duty cycle is illustrated in FIG. 9. The duty cycle represents the duration of 'on' time relative to the period. Commensurate with restoration of normal diaphragm function, a duty cycle can be modulated in a manner to reduce the need for stimulated activation of the phrenic nerve. In one example, the duration of stimulation is modulated. The duration of stimulation can represent a time period during which ultrasonic excitation is provided to the phrenic nerve.

In one example, an algorithm can be configured to select or control a frequency of the ultrasonic stimulation. The frequency of ultrasonic stimulation can be maintained at a constant or swept and in various examples, the frequency can be any frequency across a continuous spectrum from approximately 100 kHz to 1 MHz. In some examples, the frequency is 250 kHz, 300 kHz, 350 kHz, or other value. In general, the frequency will be less than 10 MHz. The excitation can be pulsed or continuous at a particular frequency (center frequency) of the transducer.

In one example, the phase of a first element of a transducer is selected or modulated relative to a phase of a second element of a transducer. In an example including multiple elements, a selectable or controllable phase allows modulation of depth of stimulation as well as controlling alignment of an axis of stimulation energy. In one example, a plurality of elements are arranged in a manner to allow the transducer to remain in a fixed position on the tissue and by modulating the phase of the various elements, the ultrasonic excitation can be swept through a range of locations or through a range of depths below the surface of the tissue.

In one example, an algorithm is configured to control or select an amplitude of the emitted ultrasonic energy. The amplitude is selected to prevent thermal effects in tissue near the site of the transducer (as well as the site of the phrenic nerve) and to achieve a neurological affect at the phrenic nerve. According to example, the amplitude is maintained at a level no greater than 720 mW/cm² Iₛₚₜₐ spatial-peak temporal-average intensity. At low intensity, or in short bursts, the ultrasonic energy can be delivered to the phrenic nerve and without generating heat or mechanical damage of biological tissue.

In one example, an algorithm is configured to control or select a pulse repetition frequency. The pulse repetition frequency corresponds to the frequency of sending the ultrasonic pulses. In various example, the pulse repetition frequency can be modulated by the user in order to obtain a desired rate of stimulation. The rate of stimulation can range from a continuous signal to a pulse repetition frequency that is significantly lower than the acoustic stimulation frequency. In this instance, the term significantly lower can be construed as at least 10 times lower, by which, for example, if the acoustic stimulation frequency is 100 kHz, then the highest PRF would be 10 kHz.

The delivered acoustic energy can be characterized by a variety of parameters. In one example, the stimulation delivers an acoustic pressure in the range of up to 5 MPa and has a waveform that can be described as a square, sinusoidal, pulsed, triangle, or arbitrary profile, and a PRF up to 100 Hz.

In various examples, ultrasonic energy can be delivered unilaterally or bilaterally. Unilateral entails delivering energy on a single side of the neck and stimulating a single phrenic nerve (left or right) and bi-lateral entails delivering energy on both sides of the neck and stimulating both left and right nerves. In some examples, at least one transducer is affixed in a position to stimulate one nerve or both nerves.

Unilateral stimulation includes at least one transducer positioned on one side of the neck to stimulate one phrenic nerve. This arrangement will stimulate either the left phrenic nerve or the right phrenic nerve, but not both nerves. Bilateral stimulation, on the other hand, includes at least two transducers positioned with one transducer on each side of the neck to stimulate both right and left phrenic nerves. Bilateral stimulation of both right and left phrenic nerves will induce bilateral contraction of both right and left diaphragm muscles at the base of the ribcage.

In various examples, the transducer is configured to emit focused ultrasonic energy in an energy dense region having a diameter of a few millimeters (approximately 5 mm). The energy can be focused by a lens disposed between an oscillating surface of the transducer and the tissue. The lens permits passage of acoustic energy and by refraction or reflection, emitted energy can be aligned with an axis, some examples of which are described elsewhere in this document. In one example, the energy is focused by a curved emitting surface. A curved emitting surface emits energy aligned substantially normal with a surface. A curved emitting surface, along with a coupling medium between the transducer and the tissue, can be configured to yield a focused emission. As described elsewhere in this document an array of phase-controlled elements can be operated in a manner to focus or align emitted energy. In one example, an aperture at the emitting surface of the transducer provides emitted energy alignment.

According to the invention, the transducer serves as both an emitter of ultrasonic energy and as a sensor. In this manner, controller 30 executes an algorithm to control operation of the transducer so that, for example, during a first duration, the transducer operates as an emitter of ultrasonic energy and during a second duration (non-overlapping with the first duration), the transducer operates as a sensor that can provide an output signal indicative of a physiological condition or parameter. For example, a transducer can be configured to detect a hiccup or other feedback signal to indicate a phrenic nerve location or indicate patient health.

The transducer can include a piezoelectric crystal configured to oscillate at a frequency and amplitude determined by an electrical signal provide on an electrical conductor coupled to the piezoelectric element. Other types of ultrasonic transducers are also contemplated, including, for example, a capacitive transducer in which an electrode plate is displaced in response to an electric current.

In one example, a system includes therapy device 15A and transducer 50A. In this example, feedback as to stimulation is not provided, or if provided, is derived from a signal received from transducer 50A or derived from visual indications or from audible detection.
System 1 of the invention includes a system for diaphragmatic pacing comprising:
   an ultrasonic transducer having an emitting surface, the emitting surface configured to couple with a tissue of a patient, the transducer configured to transdermally emit ultrasonic energy and induce movement in a thoracic diaphragm of the patient in response thereto; and
   a control module coupled to the transducer and having a processor, the processor configured to execute an algorithm to control operation of the transducer, the transducer configured to transdermally emit ultrasonic energy and stimulate a phrenic nerve of the patient, wherein the transducer is configured to provide a feedback signal to the control module and wherein the algorithm is configured to select an operational parameter for the transducer using the feedback signal, the emitted ultrasonic energy having an intensity in a range of 0.01 mW/cm2 to 1,000 W/cm2 corresponding to the operational parameter, and wherein the operational parameter corresponds to a measure of assist based on movement of the diaphragm when induced by emitted energy or based on movement of the diaphragm in the absence of ultrasonic energy.
Example 2 includes the system of System 1 wherein the algorithm is configured to control a duty cycle, a duration, a frequency, a phase, an amplitude of the emitted ultrasonic energy, or a pulse repetition frequency.
Example 3 includes the system of any one of System 1 or Example 2 wherein the transducer includes a piezoelectric element.
Example 4 includes the system of any one of System 1 and Examples 2 - 3 wherein the transducer is configured to emit energy having a peak pressure in a range of 0.01 MPa to 20 MPa, a frequency in a range of 20 kHz to 10 MHz, and a pulse width in a range of 1 microsecond to 20 seconds.
Example 5 includes the system of any one of System 1 and Examples 2 - 4 wherein the transducer includes an array of emitters.
Example 6 includes the system of any one of System 1 and Examples 2 - 5 wherein the control module is configured to control a first emitter of the array independent of a second emitter of the array.
Example 7 includes the system of any one of System 1 and Examples 2 - 6 wherein the transducer is configured to emit focused energy.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or non-volatile tangible computer-readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.

## Claims

1. A system for diaphragmatic pacing comprising:
an ultrasonic transducer (50) having an emitting surface (40), the emitting surface configured to couple with a tissue of a patient (600), the transducer configured to induce movement in a thoracic diaphragm (8) of the patient in response thereto; and
a control module (30) coupled to the transducer and having a processor, the processor configured to execute an algorithm to control operation of the transducer,
**characterized in that**
the transducer is further configured to transdermally emit ultrasonic energy and stimulate a phrenic nerve of the patient, wherein the transducer is configured to provide a feedback signal to the control module and wherein the algorithm is configured to select an operational parameter for the transducer using the feedback signal, the emitted ultrasonic energy having an intensity in a range of 0.01 mW/cm2 to 1,000 W/cm² corresponding to the operational parameter, and
wherein the operational parameter corresponds to a measure of assist based on movement of the diaphragm when induced by emitted energy or based on movement of the diaphragm in the absence of ultrasonic energy.

2. The system of claim 1 wherein the algorithm is configured to control a duty cycle, a duration, a frequency, a phase, an amplitude of the emitted ultrasonic energy, or a pulse repetition frequency.

3. The system of any one of claims 1 or 2 wherein the transducer includes a piezoelectric element.

4. The system of any one of claims 1 - 3 wherein the transducer is configured to emit energy, having a peak pressure in a range of 0.01 MPa to 20 MPa, a frequency in a range of 20 kHz to 10 MHz, and a pulse width in a range of 1 microsecond to 20 seconds.

5. The system of any one of claims 1 - 4 wherein the transducer includes an array of emitters.

6. The system of any one of claims 1 - 5 wherein the control module is configured to control a first emitter of the array independent of a second emitter of the array.

7. The system of any one of claims 1 - 6 wherein the transducer is configured to emit focused energy.

## Patentansprüche

1. System zur Zwerchfellstimulation, umfassend:
einen Ultraschallwandler (50) mit einer emittierenden Oberfläche (40), wobei die emittierende Oberfläche dazu konfiguriert ist, um mit einem Gewebe eines Patienten (600) gekoppelt zu werden, wobei der Wandler dazu konfiguriert ist, eine Bewegung in ein Zwerchfell (8) des Patienten in Reaktion hierauf zu induzieren; und
ein Steuermodul (30), das mit dem Wandler gekoppelt ist und einen Prozessor aufweist, wobei der Prozessor dazu konfiguriert ist, um einen Algorithmus auszuführen, um einen Betrieb des Wandlers zu steuern,
**dadurch gekennzeichnet, dass**
der Wandler ferner dazu konfiguriert ist, um Ultraschallenergie transdermal abzugeben und einen Zwerchfellnerv des Patienten zu stimulieren,
wobei der Wandler dazu konfiguriert ist, dem Steuermodul ein Rückkopplungssignal bereitzustellen, und wobei der Algorithmus konfiguriert ist, einen Betriebsparameter für den Wandler unter Verwendung des Rückkopplungssignals auszuwählen, wobei die abgegebene Ultraschallenergie eine Intensität in einem Bereich von 0,01 mW/cm² bis 1000 W/cm² hat, entsprechend dem Betriebsparameter, und
wobei der Betriebsparameter einer Unterstützungsmessung basierend auf einer Bewegung des Zwerchfells, wenn durch abgegebene Energie induziert, oder basierend auf einer Bewegung des Zwerchfells ohne Ultraschallenergie, entspricht.

2. System nach Anspruch 1, wobei der Algorithmus konfiguriert ist, um ein Tastverhältnis, eine Dauer, eine Frequenz, eine Phase, eine Amplitude der abgegebenen Ultraschallenergie oder eine Impulswiederholungsfrequenz zu steuern.

3. System nach Anspruch 1 oder 2, wobei der Wandler ein piezoelektrisches Element enthält.

4. System nach einem der Ansprüche 1 bis 3, wobei der Wandler konfiguriert ist, eine Energie zu emittieren, welche einen Spitzendruck in einem Bereich von 0,01 MPa bis 20 MPa, eine Frequenz in einem Bereich von 20 kHz bis 10 MHz und eine Impulsbreite in einem Bereich von 1 Mikrosekunde bis 20 Sekunden aufweist.

5. System nach einem der Ansprüche 1 bis 4, wobei der Wandler eine Anordnung von Emittern enthält.

6. System nach einem der Ansprüche 1 bis 5, wobei das Steuermodul konfiguriert ist, um einen ersten Emitter der Anordnung unabhängig von einem zweiten Emitter der Anordnung zu steuern.

7. System nach einem der Ansprüche 1 bis 6, wobei der Wandler zum Emittieren von fokussierter Energie konfiguriert ist.

## Revendications

1. Système pour stimulation d'un diaphragme, comprenant :
un transducteur à ultrasons (50) ayant une surface émettrice (40), la surface émettrice étant configurée pour être couplée avec un tissu d'un patient (600), le transducteur étant configuré pour induire un mouvement dans un diaphragme thoracique (8) du patient en réponse à celui-ci ; et
un module de commande (30) couplé au transducteur et ayant un processeur, le processeur étant configuré pour exécuter un algorithme afin de commander le fonctionnement du transducteur,
**caractérisé en ce que**
le transducteur est en outre configuré pour émettre par voie transdermique une énergie ultrasonore et stimuler un nerf phrénique du patient,
dans lequel le transducteur est configuré pour fournir un signal de rétroaction au module de commande et dans lequel l'algorithme est configuré pour sélectionner un paramètre opérationnel du transducteur en utilisant le signal de rétroaction, l'énergie ultrasonore émise ayant une intensité dans une plage de 0,01 mW/cm² à 1000 W/cm² correspondant au paramètre opérationnel, et
dans lequel le paramètre opérationnel correspond à une mesure d'assistance basée sur un mouvement du diaphragme lorsqu'il est induit par l'énergie émise ou basée sur un mouvement du diaphragme en l'absence d'énergie ultrasonore.

2. Système selon la revendication 1, dans lequel l'algorithme est configuré pour commander un cycle de service, une durée, une fréquence, une phase, une amplitude de l'énergie ultrasonore émise, ou une fréquence de répétition d'impulsion.

3. Système selon l'une quelconque des revendications 1 ou 2, dans lequel le transducteur inclut un élément piézoélectrique.

4. Système selon l'une quelconque des revendications 1 à 3, dans lequel le transducteur est configuré pour émettre de l'énergie, ayant une pression de pointe dans une plage de 0,01 MPa à 20 MPa, une fréquence dans une plage de 20 kHz à 10 MHz, et une largeur d'impulsion dans une plage de 1 µs à 20 s.

5. Système selon l'une quelconque des revendications 1 à 4, dans lequel le transducteur inclut un réseau d'émetteurs.

6. Système selon l'une quelconque des revendications 1 à 5, dans lequel le module de commande est configuré pour commander un premier émetteur du réseau indépendamment d'un second émetteur du réseau.

7. Système selon l'une quelconque des revendications 1 à 6, dans lequel le transducteur est configuré pour émettre une énergie focalisée.
